(19) Europäisches Patentamt
European Patent Office
Office européen des brevets

(11) **EP 1 748 031 A1**

(12) **EUROPEAN PATENT APPLICATION**
published in accordance with Art. 158(3) EPC

(43) Date of publication:
**31.01.2007 Bulletin 2007/05**

(51) Int Cl.:
***C01B 31/10*** (2006.01)  ***B01J 20/20*** (2006.01)
***A61K 33/44*** (2006.01)  ***A61P 1/16*** (2006.01)
***A61P 13/12*** (2006.01)

(21) Application number: **05728871.4**

(22) Date of filing: **04.04.2005**

(86) International application number:
**PCT/JP2005/006623**

(87) International publication number:
**WO 2005/095276 (13.10.2005 Gazette 2005/41)**

(84) Designated Contracting States:
**AT BE BG CH CY CZ DE DK EE ES FI FR GB GR HU IE IS IT LI LT LU MC NL PL PT RO SE SI SK TR**

(30) Priority: **02.04.2004 JP 2004110577**

(71) Applicant: **Kureha Corporation
Tokyo 103-8552 (JP)**

(72) Inventors:
• **SONOBE, Naohiro,
Research Center, Kureha Corp.
Iwaki-shi,
Fukushima 9748686 (JP)**
• **WAKAHOI, Takashi,
Reasearch Center, Kureha Corp.
Iwaki-shi,
Fukushima 9748686 (JP)**

• **KUWAHARA, Mieko,
Research Center, Kureha Corp.
Iwaki-shi,
Fukushima 9748686 (JP)**
• **OHTA, Hiroshi,
Research Center, Kureha Corp.
Iwaki-shi,
Fukushima 9748686 (JP)**

(74) Representative: **Siegert, Georg
Hoffmann - Eitle
Patent- und Rechtsanwälte
Arabellastrasse 4
81925 München (DE)**

(54) **METHOD FOR PRODUCING SPHERICAL ACTIVATED CARBON**

(57) A process for manufacturing a spherical activated carbon, **characterized by** comprising the steps of:
(1) forming a spherical substance of a heat-fusible resin,
(2) oxidizing the spherical substance of a heat-fusible resin to form a heat-infusible spherical substance, and
(3) activating the heat-infusible spherical substance to form the spherical activated carbon is disclosed. According to the process for the manufacture, a spherical activated carbon having desirable properties, such as an average particle diameter, a particle size distribution, a pore volume, or a specific surface area, can be easily prepared.

EP 1 748 031 A1

**Description**

TECHNICAL FIELD

The present invention relates to a process for manufacturing a spherical activated carbon.

BACKGROUND ART

In patients suffering a lack of a renal function or a liver function, harmful toxic substances are accumulated or formed in bodies, such as blood, with a progress of a disorder of the organ functions, and thus an encephalopathia occurs, such as a disturbance of consciousness or uremia. Yearly, there is a growing number of such patients, and therefore, the development of an organ-substitute apparatus or medicament having a function to remove toxic substances from bodies, in place of such defective organs, has become a serious problem. A method for removing toxic substances by hemodialysis as an artificial kidney is prevalent. Nevertheless, the hemodialysis-based artificial kidney requires a special apparatus, and thus, a skilled specialist is required from a safe operation standpoint. Further, blood must be taken from a patient's body, and thus, there are disadvantages in that patients must bear high physical, mental and economic burdens. Accordingly, hemodialysis is not satisfactory.

As a means of remedying the above disadvantages, an oral adsorbent which can be orally administered and cure a disorder of renal and liver functions was developed and utilized [Patent Reference No. 1]. The adsorbent disclosed in Patent Reference No. 1 comprises a porous spherical carbonaceous substance having particular functional groups, that is, a surface-modified spherical activated carbon, having a high safety factor and stable to a body, and having a useful selective adsorbability; that is, an excellent adsorbability of harmful substances in the presence of a bile acid in an intestine, and a low adsorbability of useful substances such as digestive enzymes in the intestine. For these reasons, the oral adsorbent is widely and clinically used for a patient suffering from a disorder of a liver or renal function, as an adsorbent having few side effects such as constipation. The above adsorbent disclosed in Patent Reference No. 1 was prepared by forming a spherical activated carbon from a pitch such as a petroleum pitch as a carbon source, and then carrying out an oxidizing treatment and a reducing treatment.

Further, an adsorbent for an oral administration providing an improvement in the above useful selective adsorbability, that is, an excellent adsorbability of harmful substances and a low adsorbability of useful substances in the intestine, is known (Patent Reference No. 2). The adsorbent for an oral administration disclosed in Patent Reference No. 2 is based on a finding that the above selective adsorbability is improved within a special range of a pore volume, that is, when a volume of pores having a pore diameter of 20 to 15000 nm is from not less than 0.04 mL/g to less than 0.10 mL/g. The adsorbent for an oral administration is very effective in treating diseases where a sufficient adsorption of toxins and a reduced adsorption of useful substances in the intestine are desired. The adsorbent disclosed in Patent Reference No. 2 was also prepared by forming a spherical activated carbon from a pitch such as a petroleum pitch as a carbon source, and then carrying out an oxidizing treatment and a reducing treatment.

[Patent Reference No. 1]
Japanese Examined Patent Publication (Kokoku) No. 62-11611
[Patent Reference No. 2]
Japanese Patent No. 3522708 (Japanese Unexamined Patent Publication (Kokai) No. 2002-308785)

DISCLOSURE OF THE INVENTION

PROBLEMS TO BE SOLVED BY THE INVENTION

In the surface-modified spherical activated carbon as mentioned above, it is expected that, if a pore volume, an average particle diameter, or the like varies, adsorbing properties or selective adsorbability of harmful substances are changed, and there is a possibility that a surface-modified spherical activated carbon having unknown excellent properties is developed.
In the prior art, the surface-modified spherical activated carbon as mentioned above was produced by preparing a spherical activated carbon from a pitch such as a petroleum pitch as a carbon source, and then carrying out an oxidizing treatment and a reducing treatment. In case that a spherical activated carbon is produced from a pitch as a carbon source, however, it is not necessarily easy even in the laboratory to control the properties, such as a pore volume, an average particle diameter, or the like, and it is very difficult to establish a process for large-scale production.
Accordingly, the problems of the present invention are to provide a means for facilitating a control in obtaining desired properties, such as a pore volume, an average particle diameter, or the like, when a spherical activated carbon as a raw material of the surface-modified spherical activated carbon as mentioned above is prepared.

MEANS FOR SOLVING THE PROBLEMS

The above problems can be solved according to the present invention, by the process for manufacturing a spherical activated carbon, characterized by comprising the steps of:

(1) forming a spherical substance of a heat-fusible resin,
(2) oxidizing the spherical substance of a heat-fusible resin to form a heat-infusible spherical substance, and
(3) activating the heat-infusible spherical substance to form the spherical activated carbon.

In a preferable embodiment of the present process, the spherical activated carbon wherein an average particle diameter is 0.01 to 1 mm, a specific surface area determined by a BET method is 700 $m^2$/g or more, and a volume of pores having a diameter of 7.5 to 15000 nm is 0.01 mL/g to 1 mL/g is obtained.
A preferable embodiment of the present process further comprises the step of:

oxidizing and reducing the spherical activated carbon to form a surface-modified spherical activated carbon.

In a preferable embodiment of the present process, the surface-modified spherical activated carbon wherein an average particle diameter is 0.01 to 1 mm, a specific surface area determined by a BET method is 700 $m^2$/g or more, a volume of pores having a diameter of 7.5 to 15000 nm is 0.01 mL/g to 1 mL/g, a total amount of acidic groups is 0.30 to 1.20 meq/g, and a total amount of basic groups is 0.20 to 0.90 meq/g is obtained.
In a preferable embodiment of the present process, the heat-fusible resin is cross-linked vinyl resin.
In a preferable embodiment of the present process, a specific surface area of the spherical substance of a heat-fusible resin is 10 $m^2$/g or more.
In a preferable embodiment of the present process, a content of elements other than a carbon atom, a hydrogen atom, and an oxygen atom in the heat-fusible resin is 15% by weight or less.
In a preferable embodiment of the present process, the spherical activated carbon or the surface-modified spherical activated carbon for an adsorbent for an oral administration is prepared.

EFFECTS OF THE INVENTION

According to the manufacturing process of the present invention, various properties, such as a pore volume or an average particle diameter, of the produced spherical activated carbon can be easily controlled by changing various manufacturing conditions in the manufacturing process. Therefore, a surface-modified spherical activated carbon having desired various properties, such as a pore volume or an average particle diameter, can be easily obtained by further oxidizing and reducing the resulting spherical activated carbon.

BEST MODE FOR CARRYING OUT THE INVENTION

The first step in the manufacturing process of the present invention is to form a spherical substance of a heat-fusible resin, that is, a heat-fusible resin sphere.
The term "heat-fusible resin" as used herein means a resin from which an activated carbon cannot be produced because it is melted and decomposed as a temperature is raised, if an activation treatment is carried out before a treatment to impart infusibility. However, when the heat-fusible resin is treated to impart infusibility, and then is activated, an activated carbon can be produced therefrom. The "heat-fusible resin" is the term opposite to the heat-infusible resin. The heat-infusible resin means a resin from which an activated carbon can be produced by the proceeding of carbonization (accompanying some decomposition) without melting as a temperature is raised, even if a treatment to impart infusibility is not carried out in advance. The treatment to impart infusibility is, for example, an oxidation treatment carried out at 150°C to 400°C under an atmosphere containing oxygen, as mentioned below.
A typical example of the heat-fusible resin is a thermoplastic resin, such as a cross-linked vinyl resin. A typical example of the heat-infusible resin is a thermosetting resin, such as a phenol or furan resin. Any known thermoplastic resin from which a spherical shape is formed can be used. In the present invention, the heat-fusible resin includes the thermosetting resin from which an activated carbon can be obtained by the activation after the treatment to impart infusibility, but it is melted and decomposed as a temperature is raised, if an activation treatment is carried out before a treatment to impart infusibility. Thus, any thermosetting resin which has the above property and from which a spherical shape is formed can be used. When the spherical activated carbon or the surface-modified spherical activated carbon is produced from the cross-linked vinyl resin, the above treatment to impart infusibility is necessary. On the other hand, the above treatment to impart infusibility is not necessary when the spherical activated carbon or the surface-modified spherical activated carbon is produced from an ion-exchange resin prepared by applying functional groups to the cross-linked vinyl resin. It is believed that the cross-linked resin is modified from the heat-fusible resin to the heat-infusible resin by the treatment used to introduce the functional groups thereto, and the functional groups introduced thereby. That is, the cross-linked vinyl resin belongs to the heat-fusible resin as used herein, whereas the ion-exchange resin belongs to the heat-infusible resin as used herein.

The sphere of the heat-fusible resin, such as the cross-linked vinyl resin, used as a starting material may be, for example, a spherical polymer prepared by an emulsion polymerization, a bulk polymerization, or a solution polymerization, preferably a spherical polymer prepared by a suspension polymerization. When the spherical cross-linked vinyl resin having a particle diameter of 50 $\mu$m or more is treated to uniformly impart infusibility, pores must be formed in advance in the cross-linked vinyl resin. The pores can be formed in the resin by adding porogen during the polymerization step. The surface area of the cross-linked vinyl resin required to uniformly impart infusibility thereto is preferably 10 m$^2$/g or more, more preferably 50 m$^2$/g or more.

For example, when the cross-linked vinyl resin is prepared by a suspension polymerization, an organic phase containing vinyl monomers, a cross-linking agent, porogen, and a polymerization initiator is added to an aqueous dispersion medium containing a dispersion-stabilizing agent, the whole is mixed with stirring to form many organic droplets suspended in an aqueous phase, and the monomers in the organic droplets are polymerized by heating, to thereby prepare the spherical cross-linked vinyl resin.

As the vinyl-based monomer, any vinyl-based monomer from which a spherical shape can be formed may be used. For example, an aromatic vinyl-based monomer, such as styrene, a styrene derivative wherein a hydrogen atom of a vinyl group or a phenyl group is substituted, or a compound wherein a heterocyclic or polycyclic compound is bonded to a vinyl group instead of a phenyl group can be used. An example of the aromatic vinyl-based monomer may be $\alpha$- or $\beta$-methyl styrene, $\alpha$- or $\beta$-ethyl styrene, methoxy styrene, phenyl styrene, or chlorostyrene, or, o-, m-, or p-methyl styrene, ethyl styrene, methoxy styrene, methylsilyl styrene, hydroxylstyrene, chloro-styrene, cyanostyrene, nitrostyrene, aminostyrene, carboxy-styrene, or sulfoxystyrene, sodium styrene sulfonate, or vinyl pyridine, vinyl thiophene, vinyl pyrrolidone, vinyl naphthalene, vinyl anthracene, or vinylbiphenyl. Further, an aliphatic vinyl-based monomer can be used. For example, there may be mentioned vinyl esters such as ethylene, propylene, isobutylene, diisobutylene, vinyl chloride, acrylate, methacrylate, or vinyl acetate; vinylketones such as vinyl methyl ketone, or vinyl ethyl ketone; vinylaldehydes, such as acrolein, or methacrolein; vinylethers, such as vinylmethylether, or vinylethylether; or vinyl nitriles, such as acrylonitrile, ethyl acrylonitrile, diphenyl acrylonitrile, chloroacrylonitrile.

Any cross-linking agent which may be used for the cross-lining of the above vinyl-based monomer may be used. For example, there may be mentioned divinylbenzene, divinylpyridine, divinyltoluene, divinylnaphthalene, diallyl phthalate, ethylene glycol diacrylate, ethylene glycol dimethylate, divinylxylene, divinylethylbenzene, divinylsulfone, polyvinyl or polyallyl ether of glycol or glycerol, polyvinyl or polyallyl ether of pentaerythritol, polyvinyl or polyallyl ether of mono or dithio derivative of glycol, polyvinyl or polyallyl ether of resorcinol, divinyl ketone, divinyl sulfide, allyl acrylate, diallyl maleate, diallyl fumarate, diallyl succinate, diallyl carbonate, diallyl malonate, diallyl oxalate, diallyl adipate, diallyl sebacate, triallyl tricarballylate, triallyl aconitate, triallyl citrate, triallyl phosphate, N,N'-methylene diacrylamide, 1,2-di($\alpha$-methylmethylenesulfoneamido)ethylene, trivinylbenzene, trivinylnaphthalene, polyvinylanthracene, or trivinylcyclohexane. A Particularly preferable cross-linking agent is polyvinyl aromatic hydrocarbon, such as divinylbenzene, glycol trimethacrylate such as ethylene glycol dimethacrylate, or polyvinyl hydrocarbon such as trivinyl cyclohexane). Divinylbenzene is most preferable, because of an excellent property of thermal decomposition.

As an appropriate porogen, there may be mentioned alkanol having 4 to 10 carbon atoms, such as, n-butanol, sec-butanol, 2-ethylhexanol, decanol, or 4-methyl 2-pentanol, alkyl ester having at least 7 carbon atoms, such as n-hexyl acetate, 2-ethylhexyl acetate, methyl oleate, dibutyl cebacate, dibutyl adipate, or dibutylcarbonate, alkyl ketone having 4 to 10 carbon atoms, such as dibutyl ketone or methyl isobutyl ketone, or alkyl carboxylic acid, such as heptanoic acid, aromatic hydrocarbon, such as toluene, xylene, or benzene, higher saturated aliphatic hydrocarbon, such as hexane, heptane, or isooctane, or cyclic aliphatic hydrocarbon, such as cyclohexane.

A polymerization initiator is not particularly limited, and an initiator usually used in this field can be used in the present invention. An oil soluble initiator which is soluble in a polymerizable monomer is preferable. As an example of the polymerization initiator, there may be mentioned a dialkyl peroxide, a diacyl peroxide, a peroxyester, a peroxydicarbonate, or an azo compound. More particularly, a dialkyl peroxide, such as methylethyl-peroxide, di-t-butyl peroxide, or dicumyl peroxide; a diacyl peroxide, such as isobutylperoxide, benzoylperoxide, 2,4-dichloro-benzoylperoxide, or 3,5,5-trimethylhexanoyl peroxide; a peroxyester, such as t-butylperoxypyvalate, t-hexyl-peroxypyvalate, t-butylperoxyneodecanoate, t-hexylperoxy-neodecanoate, 1-cyclohexyl 1-methylethylperoxyneodecanoate, 1,1,3,3-tetramethylbutylperoxyneodecanoate, cumyl peroxy-neodecanoate, or ($\alpha,\alpha$-bisneodecanoyl peroxy)diisopropyl-benzene; a peroxydicarbonate, such as bis(4-t-butyl-cyclohexyl)peroxy-dicarbonate, di n-propyl-oxy dicarbonate, diisopropyl peroxydicarbonate, di(2-ethylethylperoxy)-dicarbonate, dimethoxybutylperoxydicarbonate, di(3-methyl 3-methoxybutylperoxy)dicarbonate; or an azo compound, such as 2,2'-azobisisobutylonitorile, 2,2'-azobis(4-methoxy-2,4-dimethylvaleronitrile, 2,2'-azobis (2,4-dimethylvalero-nitrile), or 1,1'-azobis(1-cyclohexanecarbonitrile).

The composition of the cross-linked vinyl resin is not limited. In the manufacturing process of the present invention, however, the oxidation step and the activation step are carried out. Therefore, the cross-linked vinyl resin preferably does not contain elements such as sulfur, nitrogen, or halogen, which may possibly become a noxious gas, or an oxide of which may become a noxious gas.

Further, such elements may remain in the spherical activated carbon, and thus, influences therefrom are inestimable. Therefore, a content of elements other than a carbon atom, a hydrogen atom, and an oxygen atom in the heat-fusible resin (cross-linked vinyl resin) is preferably 15% by weight or less, more preferably 10% by weight or less, and particularly preferably 5% by weight or less.

According to the present process, various properties, such as an average particle diameter, a pore volume, a particle size distribution, or a specific surface area, of the finally resulting spherical activated carbon or surface-modified spherical activated carbon can be controlled by variously changing the manufacturing conditions in the step of forming the sphere of the heat-fusible resin. For example, the average particle diameter and the particle size distribution of the spherical activated carbon or the surface-modified spherical activated carbon varies with the size of droplet in an aqueous phase, and the size of the droplet can be controlled by an amount of a suspending agent, the number of stirring revolutions, a shape of the stirring blade, or a monomer ratio in an aqueous phase, that is, a ratio of an amount of water and an amount of monomers. For example, the size of the droplet can be lowered by increasing an amount of a suspending agent, or increasing the number of stirring revolutions. Further, it is preferable to decrease an amount of monomers in an aqueous phase, not only because an aggregation of droplets can be controlled, but also because a heat of polymerization can be easily removed. However, it is not preferable, in view of productivity, that an amount of monomer ratio is too low, because an amount of monomers per a batch, and thus, an amount of synthetic resin produced is decreased.

When the controlled pore diameter is 10 nm or more, the pore volume and the specific surface area can be controlled mainly by an amount and a kind of porogen. When the controlled pore diameter is 10 nm or less, the pore volume and the specific surface area can be controlled by conditions of steam activation. In addition, the microtexture as the spherical activated carbon or the surface-modified spherical activated carbon can be controlled by a kind of a resin, a kind and an amount of a cross-linking agent, conditions for imparting infusibility, and/or activating temperature, or the like.

It is preferable that the average particle diameter and the particle size distribution of the heat-fusible resin sphere prepared in this step are approximately equal to those of the desired spherical activated carbon or the surface-modified spherical activated carbon.

The second step in the manufacturing process of the present invention is to oxidize the heat-fusible resin sphere to form a heat-infusible spherical substance.

When the heat-fusible resin sphere is directly activated, the sphere is softened and changed to an aspherical shape, or fused together. Thus, the softening can be inhibited by an oxidation at 150°C to 400°C in an atmosphere containing oxygen, as a treatment to impart infusibility. If the temperature is too low, the oxidation would unfavorably be insufficient, and if the temperature is too high, the resin would unfavorably be decomposed. The optimal temperature at the oxidation treatment varies with the period of the oxidation treatment. A prolonged retention time has the effect same as that of an elevation of the temperature of the oxidation treatment. However, the prolonged retention time causes the lowered industrial productivity, and thus, the shorter retention time (period of oxidation treatment) is preferable. In such a sense, the temperature ultimately elevated in the oxidation treatment is preferably 230°C to 350°C, more preferably 250°C to 330°C. The degree of infusibility can be judged from a content of oxygen in the product prepared by imparting infusibility to the heat-fusible resin, i.e., the heat-infusible sphere. The oxygen content is preferably 7% to 25% by weight, more preferably 10% to 23% by weight, particularly preferably 10% to 20% by weight.

Further, if many pyrolysis gases or the like are generated by the heat-treatment of the spherical heat-fusible resin which has been treated to impart infusibility, pyrolysis products may be removed in advance by carrying out a pre-calcination, prior to the activation. If the temperature of pre-calcination is too low, pyrolysis would be insufficient. Therefore, the temperature of pre-calcination is preferably 500°C to 1000°C. The pre-calcination can be carried out in a moving bed, a fluidized bed, or a fixed bed, but the fluidized bed is preferable, because an adhesion of tar or the like to the resin or a fusion of particles is lowered.

The third step in the manufacturing process of the present invention is to activate the heat-infusible spherical substance to form the spherical activated carbon.

In the activating treatment, procedures substantially the same as a conventional method for production from pitch can be used. For example, the heat-infusible spherical substance can be activated at 700 to 1000°C in a gas stream reactive with carbon to obtain the spherical activated carbon. For example, the gas stream reactive with carbon may be prepared by diluting the reactive gas with nonreactive gas, such as nitrogen. The activating rate varies with a composition, a concentration or a temperature of gas used. For example, when steam is used, the reaction commences at about 700°C, but the reaction rate is very slow. When the temperature is 1000°C or more, the reaction rate becomes high, and thus, a diffusion rate of the reaction gas is a rate determining step of the activation and the control of the activation degree, whereby a good pore structure is not obtained. The reason that the diffusion rate of the reaction gas becomes a rate determining step is that the reaction rate becomes rapid more than the rate of the gas diffusing into the inside of the particle, and thus, the activation reaction occurs dominantly on the particle surface. Therefore, the activating temperature is more preferably 760°C to 1000°C, particularly preferably 800°C to 1000°C. The activation can be carried out in a moving bed, a fluidized bed, or a fixed bed, but the fluidized bed is preferable, because a temperature distribution in a reaction system is narrow and thus, a homogeneous activation is facilitated, a relatively large amount of the reactive gas is easily charged into the reaction system, and the spherical material easily and thus, homogenously flows.

As mentioned above, in the desired spherical activated carbon or surface-modified spherical activated carbon, the pore structure having a pore diameter of 10 nm or less, particularly 3 nm or less can be adjusted by controlling the activation degree. Specifically, micropores are formed in an initial stage of the activation, and then, the micropores are changed to pores having a larger pore diameter, as the activation proceeds.

The term "activated carbon" as used herein means a porous product prepared by a heat-treatment of a carbon precursor such as a spherical heat-infusible resin, and a subsequent activation, and the term "spherical activated carbon" as used herein means an activated carbon having a spherical shape and a specific surface area of 100 $m^2/g$ or more.

According to the present process comprising the above first step to the third step, for example, a spherical activated carbon wherein an average particle diameter is 0.01 to 1 mm, a specific surface area determined by a BET method is 700 $m^2/g$ or more, and a volume of pores having a pore diameter of 7.5 to 15000 nm is 0.01 mL/g to 1 mL/g can be prepared. That is, a spherical activated carbon wherein the average particle diameter is any value within the range of 0.01 to 1mm, for example, 40 to 1000$\mu$m, 40 to 600$\mu$m, or 50 to 200$\mu$m, the specific surface area determined by a BET method is any value within the range of 700 $m^2/g$ or more, for example, 700 to 3000 $m^2/g$, 1100 to 2500 $m^2/g$, or 1300 to 2500 $m^2/g$, and the volume of pores having a pore diameter of 7.5 to 15000 nm is any value within the range of 0.01 mL/g to 1 mL/g, for example, 0.01 to 0.5 mL/g, 0.01 to 0.25 mL/g, or 0.01 to 0.1 mL/g, can be prepared.

In the present invention, a step of oxidizing and reducing the above spherical activated carbon to form a surface-modified spherical activated carbon can be further carried out as a fourth step following the third step.

The spherical activated carbon is oxidized at 300 to 800°C, preferably 320 to 600°C, in an atmosphere containing 0.1 to 50 vol%, preferably 1 to 30 vol%, particularly preferably 3 to 20 vol% of oxygen, and then reduced at 800 to 1200°C, preferably 800 to 1000°C, in an atmosphere of non-oxidative gas, to thereby obtain the surface-modified spherical activated carbon. Pure oxygen, nitrogen oxide, air, or the like can be used as an oxygen source in the particular atmosphere containing oxygen. The atmosphere inactive to carbon means nitrogen, argon, or helium, alone or a mixture thereof.

The term "surface-modified spherical activated carbon" as used herein means a porous product prepared by the oxidizing and reducing treatments of the spherical activated carbon as above, wherein acidic and basic sites are added in a well-balanced manner on the surface of the spherical activated carbon to thereby improve an adsorbability of harmful substances. For example, specificity to or selective adsorbability of harmful substances to be adsorbed can be enhanced by the oxidizing and reducing treatments of the spherical activated carbon as above.

According to the present process comprising the above first step to the fourth step, for example, a surface-modified spherical activated carbon wherein an average particle diameter is 0.01 to 1 mm, a specific surface area determined by a BET method is 700 $m^2/g$ or more, a volume of pores having a pore diameter of 7.5 to 15000 nm is 0.01 mL/g to 1 mL/g, a total amount of acidic groups is 0.30 to 1.20 meq/g, and a total amount of basic groups is 0.20 to 0.90 meq/g, can be prepared. That is, a spherical activated carbon wherein the average particle diameter is any value within the range of 0.01 to 1mm, for example, 30 to 1000$\mu$m, 40 to 600$\mu$m, or 50 to 200$\mu$m, the specific surface area determined by a BET method is any value within the range of 700 $m^2/g$ or more, for example, 700 to 3000 $m^2/g$, 1100 to 2500 $m^2/g$, or 1300 to 2500 $m^2/g$, and the volume of pores having a pore diameter of 7.5 to 15000 nm is any value within the range of 0.01 mL/g to 1 mL/g, for example, 0.01 to 0.5 mL/g, 0.01 to 0.25 mL/g, or 0.01 to 0.1 mL/g, the total amount of acidic groups is any value within the range of 0.30 to 1.20 meq/g, for example, 0.30 to 1.00 meq/g, 0.30 to 0.80 meq/g, or 0.40 to 0.70 meq/g, and the total amount of basic groups is any value within the range of 0.20 to 0.90 meq/g, for example, 0.30 to 0.80 meq/g, 0.40 to 0.80 meq/g, or 0.40 to 0.70 meq/g, can be prepared.

Properties of the spherical activated carbon or the surface-modified spherical activated carbon prepared by the process of the present invention, namely, the average particle diameter, the specific surface area, the pore volume, the particle size distribution, the total amount of acidic groups, and the total amount of basic groups, are measured by the following methods.

(1) An average particle diameter (Dv50)

A particle-sizes accumulating standard curve with respect to a volume basis is prepared by a laser diffraction apparatus for measuring particle size distribution [SALAD-3000S; Shimadzu Corporation]. A particle size at a particle-sizes accumulating ratio of 50% is determined as an average particle diameter (Dv50).

(2) A bulk density

This is measured in accordance with a method for measuring a packing density defined in JIS K 1474-5.7.2.

(3) A specific surface area (method for calculating a specific surface area by a BET method)

An amount of gas adsorbed is measured by a specific surface area measuring apparatus (for example, ASAP2010 manufactured by MICROMERITICS) in accordance with a gas adsorbing method for the spherical activated carbon sample or the surface-modified spherical activated carbon sample, and a specific surface area can be calculated by the following adsorption equation. More particularly, the spherical activated carbon or the surface-modified spherical activated carbon is charged as a sample in a sample tube, and dried under a reduced pressure at 300°C. Thereafter, a weight of a dried sample is measured. Then, the test tube is cooled to -196°C, and nitrogen is introduced into the test tube, whereby nitrogen is adsorbed to the spherical activated carbon sample or the surface-modified spherical activated carbon sample. A relation of a nitrogen partial pressure and an adsorbed amount (absorption-isotherm line) is measured. BET plotting is carried out, given that a relative pressure of nitrogen is p, and an adsorbed amount at that time is v(cm$^3$/g STP). That is, the plotting in a range wherein p is 0.02 to 0.20 is carried out, in the field wherein a longitudinal axis is p/(v(1-p)), and an abscissa axis is p. Given that the gradient at that time is b(g/cm$^3$) and an intercept is c(g/cm$^3$), a specific surface area S (unit = m$^2$/g) can be calculated from the equation:

$$[\text{equation 1}]$$
$$S = [MA \times (6.02 \times 10^{23})]/[22414 \times 10^{18} \times (b+c)]$$

wherein MA denotes a cross-sectional area of a nitrogen molecule, and is 0.162 nm$^2$.

(4) A specific surface area (method for calculating a specific surface area by a Langmuir's equation)

An amount of gas adsorbed is measured by a specific surface area measuring apparatus (for example, ASAP2010 manufactured by MICROMERITICS) in accordance with a gas adsorbing method for the spherical activated carbon sample or the surface-modified spherical activated carbon sample, and a specific surface area can be calculated by Langmuir's adsorption equation. More particularly, the spherical activated carbon or the surface-modified spherical activated carbon is charged as a sample in a sample tube, and dried under a reduced pressure at 300°C. Thereafter, a weight of a dried sample is measured. Then, the test tube is cooled to -196°C, and nitrogen is introduced into the test tube, whereby nitrogen is adsorbed to the spherical activated carbon sample or the surface-modified spherical activated carbon sample. A relation of a nitrogen partial pressure and an adsorbed amount (absorption-isotherm line) is measured. Langmuir's plotting is carried out, given that a relative pressure of nitrogen is p, and an adsorbed amount at that time is v (cm$^3$/g STP). That is, the plotting in a range wherein p is 0.02 to 0.20 is carried out, in the field wherein a longitudinal axis is p/v, and an abscissa axis is p. Given that the gradient at that time is b(g/cm$^3$), a specific surface area S (unit = m$^2$/g) can be calculated from the equation:

$$[\text{equation 2}]$$
$$S = [MA \times (6.02 \times 10^{23})]/[22414 \times 10^{18} \times b]$$

wherein MA denotes a cross-sectional area of a nitrogen molecule, and is 0.162 nm$^2$.

(5) A pore volume by a mercury injection method

The pore volume can be measured by a mercury porosimeter (for example, AUTOPORE 9200 manufactured by MICROMERITICS). The spherical activated carbon or the surface-modified spherical activated carbon is charged as a sample in a sample vessel, and degassed under a pressure of 2.67Pa or less for 30 minutes. Then, mercury is introduced into the sample vessel, a pressure applied is gradually increased (maximum pressure = 414 MPa) to force the mercury into the micropores in the spherical activated carbon sample or the surface-modified spherical activated carbon sample. A pore volume distribution of the spherical activated carbon sample or the surface-modified spherical activated carbon sample is measured from a relationship between the pressure and an amount of forced mercury, by equations as mentioned below.

Specifically, a volume of mercury inserted into the spherical activated carbon sample or the surface-modified spherical activated carbon sample while a pressure is applied is increased from a pressure (0.06 MPa) corresponding to a pore diameter of 21 $\mu$m to the maximum pressure (414 MPa) corresponding to a pore diameter of 3 nm. A pore diameter can be calculated as follows. When mercury is forced into a cylindrical micropore having a diameter (D) by applying a pressure (P), a surface tension (y) of mercury is balanced with a pressure acting on a section of the micropore, and thus, a following equation is held:

$$-\pi D \gamma \cos\theta = \pi (D/2)^2 \cdot P$$

wherein $\theta$ is a contact angle of mercury and a wall of the micropore. Therefore, a following equation:

$$D = (-4\gamma\cos\theta)/P$$

is held.

In the present specification, the relationship between the pressure (P) and the pore diameter (D) is calculated by an equation:

$$D = 1.27/P$$

given that a surface tension of mercury is 484 dyne/cm, a contact angle of mercury and carbon is 130°, a unit of the pressure P is MPa, and a unit of the pore diameter D is $\mu$m. The volume of pores having a pore diameter of 7.5 to 15000 nm in the present invention corresponds to a volume of mercury inserted by applying a pressure increasing from 0.085 MPa to 169 MPa.

(6) Particle size distribution

A number-based particle distribution is measured by a laser diffraction apparatus for measuring particle size distribution [SALAD-3000S; Shimadzu Corporation] and a representative particle size D and the number n in a fraction of particles having particle size to be measured are determined. A length average particle diameter $D_1$, and a weight average particle diameter $D_4$ were calculated by the following equations:

$$[\text{equation 3}]$$
$$D_1 = \sum(nD)/\sum n$$

$$[\text{equation 4}]$$
$$D_4 = \sum(nD^4)/\sum(nD^3)$$

(7) Total amount of acidic groups

The total amount of acidic groups is an amount of NaOH consumed, which may be determined by adding 1 g of the spherical activated carbon sample or the surface-modified spherical activated carbon sample, after being crushed to form particles having a size of 200 mesh or less, to 50 mL of a 0.05N NaOH solution, shaking the mixture for 48 hours, then filtering out the spherical activated carbon sample or the surface-modified spherical activated carbon sample, and titrating until neutralization.

(8) Total amount of basic groups

The total amount of basic groups is an amount of HCl consumed, which may be determined by adding 1 g of the spherical activated carbon sample or the surface-modified spherical activated carbon sample after being crushed to form particles having a size of 200 mesh or less, to 50 mL of a 0.05N HCl solution, shaking the mixture for 24 hours, then filtering out the spherical activated carbon sample or the surface-modified spherical activated carbon sample, and titrating until neutralization.

The spherical activated carbon which can be prepared by the process of the present invention, or particularly, the surface-modified spherical activated carbon which can be obtained from the above spherical activated carbon exhibits an excellent adsorbability of exacerbation factors of liver diseases or harmful substances of renal diseases, and therefore, may be used as an adsorbent for an oral administration for treating or preventing a renal disease or a liver disease.

As the renal disease, there may be mentioned, for example, chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, or hypertension syndrome, or secondary renal diseases caused by these primary diseases, or a light renal failure before a dialysis therapy, and may be used in an improvement of a light renal failure before a dialysis therapy or a disease condition for a patient during a dialysis therapy (see "Clinical Nephrology", Asakura-shoten, Nishio Honda, Kenkichi Koiso, and Kiyoshi Kurokawa, 1990; and "Nephrology" Igaku-shoin, Teruo Omae and Sei Fujimi, ed., 1981).

As the liver disease, there may be mentioned, for example, fulminant hepatitis, chronic hepatitis, viral hepatitis, alcoholic hepatitis, hepatic fibrosis, liver cirrhosis, hepatic cancer, autoimmune hepatitis, drug allergic hepatopathy, primary biliary cirrhosis, tremor, encephalopathia, dysbolism, or dysfunction. Further, the porous spherical carbonaceous substance can be used in a treatment of a disease caused by toxic substances in a body, such as psychosis.

EXAMPLES

The present invention now will be further illustrated by, but is by no means limited to, the following Examples.

Example 1

Deionized water (220 g) and methyl cellulose (58 g) were charged into a 1L separable flask. Further, 105 g of styrene, 184 g of divinyl benzene with a purity of 57% (57% divinylbenzene and 43% ethylvinyl benzene), 1.68 g of 2,2'-azobis(2,4-dimethylvaleronitrile), and 63 g of 1-butanol as a porogen were added thereto. Then, a replacement with a nitrogen gas was carried out. The two-phase system was stirred at 200 rpm, and heated to 55°C, and then allowed to stand for 20 hours. The resulting resin was filtered, and dried in a rotary evaporator. In a vacuum dryer, 1-butanol was removed from the resin by distillation, and the product was dried under a reduced pressure at 90°C for 12 hours to thereby obtain a spherical porous synthetic resin having an average particle diameter of 180 $\mu$m. A specific surface area of the porous synthetic resin was about 90 m$^2$/g.

The resulting spherical porous synthetic resin (100 g) was charged into a reactor having a grating, and treated to impart infusibility in a vertical tubular furnace. The infusibility-imparting treatment was carried out under the conditions that dried air (3 L/min) was upwardly passed from the lower portion of the reactor tube, the temperature was raised to 260°C at a rate of 5°C/h, and the whole was allowed to stand at 260°C for 4 hours to thereby obtain a spherical porous oxidized resin. The resulting spherical porous oxidized resin was heat-treated at 600°C for 1 hour under a nitrogen atmosphere, and then activated in a fluidized bed at 820°C for 10 hours under a nitrogen gas atmosphere containing 64.5% by volume of steam, to obtain a spherical activated carbon. The properties of the resulting spherical activated carbon are shown in Table 1.

Then, the resulting spherical activated carbon was oxidized in the fluidized bed at 470°C for 195 minutes under a nitrogen-oxygen atmosphere containing 18.5% by volume of oxygen, and reduced in the fluidized bed at 900°C for 17 minutes under a nitrogen gas atmosphere, to obtain a surface-modified spherical activated carbon. The properties of the resulting surface-modified spherical activated carbon are listed in Table 2.

Example 2

The procedures of Example 1 were repeated except that the two-phase system was stirred at 100 rpm, instead of 200 rpm, to obtain a spherical activated carbon and a surface-modified spherical activated carbon. The properties of the resulting spherical activated carbon are listed in Table 1, and the properties of the surface-modified spherical activated carbon are listed in Table 2.

Example 3

The procedures of Example 1 were repeated except that the two-phase system was stirred at 150 rpm, instead of 200 rpm, to obtain a spherical activated carbon and a surface-modified spherical activated carbon. The properties of the resulting spherical activated carbon are listed in Table 1, and the properties of the surface-modified spherical activated carbon are listed in Table 2.

Example 4

The procedures of Example 1 were repeated except that the two-phase system was stirred at 300 rpm, instead of 200 rpm, to obtain a spherical activated carbon and a surface-modified spherical activated carbon. The properties of the resulting spherical activated carbon are listed in Table 1, and the properties of the surface-modified spherical activated carbon are listed in Table 2.

Example 5

The procedures of Example 1 were repeated except that the activation was carried out for 6 hours, instead of 10 hours, to obtain a spherical activated carbon and a surface-modified spherical activated carbon. The properties of the resulting spherical activated carbon are listed in Table 1, and the properties of the surface-modified spherical activated carbon are listed in Table 2.

Example 6

The procedures of Example 1 were repeated except that the activation was carried out for 13 hours, instead of 10 hours, to obtain a spherical activated carbon and a surface-modified spherical activated carbon. The properties of the resulting spherical activated carbon are listed in Table 1, and the properties of the surface-modified spherical activated carbon are listed in Table 2.

[Method for evaluation of the spherical activated carbon and the surface-modified spherical activated carbon]

The properties shown in the following Table 1 (spherical activated carbon) and Table 2 (surface-modified spherical activated carbon) were measured by the following methods.

(1) Average particle diameter

The laser diffraction apparatus for measuring particle size distribution as mentioned above was used for the measuring.

(2) Pore volume

The spherical activated carbon or the surface-modified spherical activated carbon prepared in Examples 1 to 5 and Comparative Examples 1 to 4 was measured by the mercury injection method as mentioned above.

(3) Specific surface area by BET or Langmuir's method

The BET or Langmuir's method as mentioned above was used for the measuring.

(4) Bulk density

The sample was charged into a 50 mL graduated measuring cylinder until the sample reached a scale of 50 mL. After the cylinder was tapped 50 times, a weight of the sample was divided by a volume of the sample to find a bulk density. The results are shown in Tables 1 and 3. It was confirmed that the results obtained by the above method were equal to those obtained by the method for determining a packing density in accordance with JIS K 1474-5.7.2 in the range of the significant figures shown in Tables 1 and 2.

(5) Total amount of acidic groups and total amount of basic groups

The surface-modified spherical activated carbon sample (1 g), which comprised particles having a size of 200 mesh or less prepared by crushing, was added to 50 mL of a 0.05N NaOH solution (total amount of acidic groups) or 50 mL of a 0.05N HCl solution (total amount of basic groups). After the mixture was shaken for 48 hours, the surface-modified spherical activated carbon sample was filtered out, and titrated until neutralization to find an amount of NaOH consumed (total amount of acidic groups) or an amount of HCl consumed (total amount of basic groups). The results are shown in Table 2.

Table 1

| | | Average particle diameter | | | $D_4/D_1$ | Specific surface area | | Bulk density (g/mL) |
|---|---|---|---|---|---|---|---|---|
| | | Dv50 ($\mu$m) | Length $D_1$ ($\mu$m) | Weight $D_4$ ($\mu$m) | | Langmuir method ($m^2/g$) | BET method ($m^2/g$) | |
| Example 1 | Cross-linked vinyl resin | 117 | 118 | 133 | 1.12 | 2407 | 1906 | 0.50 |
| Example 2 | Cross-linked vinyl resin | 198 | 168 | 196 | 1.16 | 2451 | 1976 | 0.50 |
| Example 3 | Cross-linked vinyl resin | 150 | 142 | 156 | 1.09 | 2380 | 1921 | 0.50 |
| Example 4 | Cross-linked vinyl resin | 70 | 67 | 74 | 1.11 | 2422 | 1955 | 0.50 |
| Example 5 | Cross-linked vinyl resin | 119 | 119 | 135 | 1.14 | 1443 | 1177 | 0.63 |
| Example 6 | Cross-linked vinyl resin | 117 | 128 | 132 | 1.03 | 2715 | 2210 | 0.47 |

Table 2

| | | Average particle diameter | | | $D_4/D_1$ | Specific surface area | | Bulk density (g/mL) | Total acidic groups (meq/g) | Total basic groups (meq/g) |
|---|---|---|---|---|---|---|---|---|---|---|
| | | Dv50 ($\mu$m) | Length $D_1$ ($\mu$m) | Weight $D_4$ ($\mu$m) | | Langmuir method ($m^2$/g) | BET method ($m^2$/g) | | | |
| Example 1 | Cross-linked vinyl resin | 111 | 104 | 129 | 1.24 | 2147 | 1763 | 0.50 | 0.59 | 0.61 |
| Example 2 | Cross-linked vinyl resin | 168 | 161 | 184 | 1.14 | 2206 | 1809 | 0.50 | 0.60 | 0.58 |
| Example 3 | Cross-linked vinyl resin | 131 | 131 | 144 | 1.10 | 2142 | 1756 | 0.50 | 0.62 | 0.63 |
| Example 4 | Cross-linked vinyl resin | 63 | 62 | 69 | 1.11 | 2180 | 1788 | 0.50 | 0.55 | 0.60 |
| Example 5 | Cross-linked vinyl resin | 93 | 95 | 105 | 1.10 | 1614 | 1282 | 0.63 | 0.53 | 0.57 |
| Example 6 | Cross-linked vinyl resin | 100 | 101 | 114 | 1.13 | 2259 | 1858 | 0.47 | 0.65 | 0.60 |

EP 1 748 031 A1

INDUSTRIAL APPLICABILITY

According to the process of the present invention, the spherical activated carbon having desired properties, such as the average particle diameter, the particle size distribution, the pore volume, or the specific surface area, can be easily obtained. Further, from the above spherical activated carbon, the surface-modified spherical activated carbon having desired properties, such as the average particle diameter, the particle size distribution, the pore volume, or the specific surface area, can be easily obtained.

Although the present invention has been described with reference to specific embodiments, various changes and modifications obvious to those skilled in the art are possible without departing from the scope of the appended claims.

**Claims**

1. A process for manufacturing a spherical activated carbon, **characterized by** comprising the steps of:

   (1) forming a spherical substance of a heat-fusible resin,
   (2) oxidizing the spherical substance of a heat-fusible resin to form a heat-infusible spherical substance, and
   (3) activating the heat-infusible spherical substance to form the spherical activated carbon.

2. The process according to claim 1, for manufacturing the spherical activated carbon wherein an average particle diameter is 0.01 to 1 mm, a specific surface area determined by a BET method is 700 $m^2/g$ or more, and a volume of pores having a diameter of 7.5 to 15000 nm is 0.01 mL/g to 1 mL/g.

3. The process according to claim 1, further comprising the step of:

   oxidizing and reducing the spherical activated carbon to form a surface-modified spherical activated carbon.

4. The process according to claim 3, for manufacturing the surface-modified spherical activated carbon wherein an average particle diameter is 0.01 to 1 mm, a specific surface area determined by a BET method is 700 $m^2/g$ or more, a volume of pores having a diameter of 7.5 to 15000 nm is 0.01 mL/g to 1 mL/g, a total amount of acidic groups is 0.30 to 1.20 meq/g, and a total amount of basic groups is 0.20 to 0.90 meq/g.

5. The process according to any one of claims 1 to 4, wherein the heat-fusible resin is cross-linked vinyl resin.

6. The process according to any one of claims 1 to 5, wherein a specific surface area of the spherical substance of a heat-fusible resin is 10 $m^2/g$ or more.

7. The process according to any one of claims 1 to 6, wherein a content of elements other than a carbon atom, a hydrogen atom, and an oxygen atom in the heat-fusible resin is 15% by weight or less.

8. The process according to any one of claims 1 to 7, wherein the spherical activated carbon or the surface-modified spherical activated carbon for an adsorbent for an oral administration is prepared.

## INTERNATIONAL SEARCH REPORT

| | International application No. |
|---|---|
| | PCT/JP2005/006623 |

**A. CLASSIFICATION OF SUBJECT MATTER**
Int.Cl⁷ C01B31/10, B01J20/20//A61K33/44, A61P1/16, 13/12

According to International Patent Classification (IPC) or to both national classification and IPC

**B. FIELDS SEARCHED**

Minimum documentation searched (classification system followed by classification symbols)
Int.Cl⁷ C01B31/10, B01J20/20//A61K33/44, A61P1/16, 13/12

Documentation searched other than minimum documentation to the extent that such documents are included in the fields searched
Jitsuyo Shinan Koho 1922-1996 Jitsuyo Shinan Toroku Koho 1996-2005
Kokai Jitsuyo Shinan Koho 1971-2005 Toroku Jitsuyo Shinan Koho 1994-2005

Electronic data base consulted during the international search (name of data base and, where practicable, search terms used)
JSTPlus(JOIS)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2002-308785 A (Kureha Chemical Industry Co., Ltd.),<br>23 October, 2002 (23.10.02),<br>Claims; Par. No. [0013]; example 1<br>& EP 1249241 A1 & US 2002/176840 A1<br>& US 2003/118581 A1 & KR 2004032320 A<br>& CA 2433368 A1 | 1-5,8<br>6,7 |
| Y<br>A | JP 53-50088 A (Sumitomo Chemical Co., Ltd.),<br>08 May, 1978 (08.05.78),<br>Full text<br>(Family: none) | 1-5,8<br>6,7 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 01 July, 2005 (01.07.05) | 19 July, 2005 (19.07.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62011611 B
- JP 3522708 B
- JP 2002308785 A

**Non-patent literature cited in the description**

- **NISHIO HONDA ; KENKICHI KOISO ; KIYOSHI KUROKAWA.** Clinical Nephrology. Asakura-shoten, 1990
- Nephrology. Igaku-shoin, 1981